Europäisches Patentamt

(19) European Patent Office

Office européen des brevets

(11) Publication number : **0 500 224 A2**

# (12) EUROPEAN PATENT APPLICATION

(21) Application number : 92300752.0

(51) Int. Cl.⁵ : **C12Q 1/68**

(22) Date of filing : 29.01.92

(30) Priority : **31.01.91 US 648704**

(43) Date of publication of application :
**26.08.92 Bulletin 92/35**

(84) Designated Contracting States :
**AT BE CH DE DK ES FR GB GR IT LI LU NL PT SE**

(72) Inventor : **Walker, George T.**
**209 Mt. Bolus Road**
**Chapel Hill, NC 27514 (US)**
Inventor : **Schram, James L.**
**102 Dwelling Place**
**Knightdale, NC 27545 (US)**

(74) Representative : **Ruffles, Graham Keith et al**
**MARKS & CLERK 57-60 Lincoln's Inn Fields**
**London WC2A 3LS (GB)**

(71) Applicant : **Becton Dickinson and Company**
**One Becton Drive**
**Franklin Lakes New Jersey 07417-1880 (US)**

(54) **Exonuclease mediated strand displacement amplification.**

(57)   This invention relates a nucleic acid target amplification method which operates at a single temperature and makes use of a polymerase in conjunction with an exonuclease that will digest the primer portion of the polymerized strand such that the polymerase will displace the partially digested strand without digestion of the target strand while generating a newly polymerized strand.

EP 0 500 224 A2

## Field of the Invention

This invention relates to a method for amplifying a target nucleic acid sequence, and more particularly relates to a method for amplification by exonuclease mediated strand displacement.

## Background of the Invention

Nucleic acids may be either in the form of deoxyribonucleic acids (DNA) or in the form of ribonucleic acids (RNA). DNA and RNA are high molecular weight polymers formed from many nucleotide building blocks. Each nucleotide is composed of a base (a purine or a pyrimidine), a sugar (either ribose or deoxyribose) and a molecule of phosphoric acid. DNA is composed of the sugar deoxyribose and the bases adenine (A), guanine (G), cytosine (C) and thymine (T).

The nucleotides are assembled into a linear chain to form the genetic code. Each sequence of three nucleotides can be "read" as the code for one amino acid through the process of translation. (DNA must first be converted into RNA through the process of transcription.) By varying the combination of bases in each three base sequence, different amino acids are coded for. By linking various three base sequences together, a sequence of amino acids can be made which form proteins. The entire coding unit for one protein is referred to as a gene. There can be one or more copies of a gene in an organism. Some genes are present in hundreds or thousands of copies. Others are present only as a single copy.

Regardless of the number of copies, genes are linked together in an organism to form higher structural units referred to as chromosomes in higher organisms. In some lower organisms, genes may occur in extra chromosomal units referred to as plasmids. Genes need not be linked directly to each other in an end-to-end fashion. Certain non-coding regions (i.e., sequences of bases that do not translate into amino acids) may occur between genes or within a gene. Thus, the arrangement of nucleotides in an organism determines its genetic makeup which may be referred to as its genome. (Hence, DNA isolated from an organism is referred to as genomic DNA.)

DNA in most organisms is arranged in the form of a duplex wherein two strands of DNA are paired together in the familiar double helix. In this model, hydrogen bonds are formed between A and T and between C and G on the paired strands. Thus, on one strand, the sequence ATCG ($5' \rightarrow 3'$) will have on its complimentary strand the sequence TAGC ($3' \rightarrow 5'$). Both strands, however, contain the same genetic code only in a complementary base-paired manner. One could read, therefore, either strand of DNA in order to determine the genetic sequence coded for.

For a further description of the organization, structure and function of nucleic acids, see Watson, Molecular Biology of the Gene, W.J. Benjamin, Inc. (3rd edit. 1977), especially ch.s 6-14.

Understanding and determining the genetic sequence of nucleic acids present in a sample is important for many reasons. First, a number of diseases are genetic in the sense that the nucleotide sequence for a "normal" gene is in some manner changed. Such a change could arise by the substitution of one base for another. Given that three bases code for a single amino acid, a change in one base (referred to as a point mutation) could result in a change in the amino acid which, in turn, could result in a defective protein being made in a cell. Sickle cell anemia is a classic example of such a genetic defect caused by a change in a single base in a single gene. Other examples of diseases caused by single gene defects include Factor IX and Factor VIII deficiency, adenosine deaminase deficiency, purine nucleotide phosphorylase deficiency, ornithine transcarbamylase deficiency, argininsuccinate synthetase deficiency, beta-thalassemia, $\alpha_1$ antitrypsin deficiency, glucocerebrosidase deficiency, phenylalanine hydroxylase deficiency and hypoxanthine-guanine phosphoribosyltransferase deficiency. Still other diseases, such as cancers, are believed to be caused by the activation, translocation, transposition, increase in copy number and/or removal of suppression of genes known to be present in the genome referred to as oncogenes. Examples of oncogenes believed to be relevant to certain cancers include N-myc for neuroblastomas, retinoblastomas and small-cell lung cancers and c-abl for chronic myelogenous leukemia. For a further description of the relevance of oncogenes to the diagnosis of cancers and for a listing of specific oncogenes, see Weinberg, Sci. Amer., Nov. 1983, Slamon et al., Science, 224:256 (1984), U.S. Pat. No. 4,699,877 and 4,918,162.

Second, in addition to changes in the sequence of nucleic acids, there are genetic changes that occur on a structural level. Such changes include insertions, deletions and translocations along a chromosome and include increased or decreased numbers of chromosomes. In the former instance, such changes can result from events referred to as crossing-over where strands of DNA from one chromosome exchange various lengths of DNA with another chromosome. Thus, for example, in a "normal" individual, the gene for protein "X" might reside on chromosome 1; after a crossing-over event, that gene could now have been translocated to chromosome 4 (with or without an equal exchange of DNA from chromosome 4 to chromosome 1) and the cell may not produce X.

In the instance of increased or decreased chromosome number (referred to as aneuploidy), instead of a "normal" individual having the correct number of copies of each chromosome (e.g., two of each in humans [other than the X and Y chromosomes]), a different number occurs. In humans, for example, Down's syndrome is the result of having three copies of chromosome 21 instead of the normal two copies. Other aneuploid conditions result from trisomies involving chromosomes 13 and 18.

Third, infectious diseases can be caused by parasites, microorganisms and viruses all of which have their own nucleic acids. The presence of these organisms in a sample of biological material often is determined by a number of traditional methods (e.g., culture). Because each organism has its own genome, however, if there are genes or sequences of nucleic acids that are specific to a single species (to several related species, to a genus or to a higher level of relationship), the genome will provide a "fingerprint" for that organism (or species, etc.). Examples of viruses to which this invention is applicable include HIV, HPV, EBV, HSV, Hepatitis B and C and CMV. Examples of microorganisms to which this invention is applicable include bacteria and more particularly include H. influenzae, mycoplasma, legionella, mycobacteria, chlamydia, candida, gonocci, shigella and salmonella.

In each example set forth above, by identifying a sequence that is specific for a disease or organism, one can isolate nucleic acids from a sample and determine if that sequence is present. A number of methods have been developed in an attempt to do this.

While it is critical that one or more sequences specific for a disease or organism be identified, it is not important to the practice of this invention what the target sequences are or how they are identified. The most straightforward means to detect the presence of a target sequence in a sample of nucleic acids is to synthesize a probe sequence complementary to the target nucleic acid. (Instrumentation, such as the Applied BioSystems 380B, are presently used to synthesize relatively short nucleic acid sequences for this purpose.) The synthesized probe sequence then can be applied to a sample containing nucleic acids and, if the target sequence if present, the probe will bind to it to form a reaction product. In the absence of a target sequence and barring non-specific binding, no reaction product will be formed. If the synthesized probe is tagged with a detectable label, the reaction product can be detected by measuring the amount of label present. Southern blotting is one example where this method is used.

A difficulty with this approach, however, is that it is not readily applicable to those instances where the number of copies of the target sequence present in a sample is low (i.e., less than $10^7$). In such instances, it is difficult to distinguish signal from noise (i.e., true binding between probe and target sequences from non-specific binding between probe and non-target sequences). One way around this problem is to increase the signal. Accordingly, a number of methods have been described to amplify the target sequences present in a sample.

One of the best known amplification methods is the polymerase chain reaction (referred to as PCR) which is described in detail in U.S. Pat. No.s 4,683,195, 4,683,202 and 4,800,159. Briefly, in PCR, two primer sequences are prepared which are complementary to regions on opposite complementary strands of the target sequence. An excess of deoxynucleosidetriphosphates are added to a reaction mixture along with a DNA polymerase (e.g., Taq polymerase). If the target sequence is present in a sample, the primers will bind to the target and the polymerase will cause the primers to be extended along the target sequence by adding on nucleotides. By raising and lowering the temperature of the ,reaction mixture, the extended primers will dissociate from the target to form a reaction products, excess primers will bind to the target and to the reaction products, and the process is repeated.

Another method for amplification is described in EPA No. 320,308, published June 14, 1989, which is the ligase chain reaction (referred to as LCR). In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Pat. No. 4,883,750 describes a method similar to LCR for binding probe pairs to a target sequence but does not describe an amplification step.

A still further amplification method is described in PCT Appl. No. PCT/US87/00880, published October 22, 1987, and is referred to as the Qbeta Replicase method. In this method, a replicative sequence of RNA which has a region complementary to that of a target is added to a sample in the presence of an RNA polymerase. The polymerase will copy the replicative sequence which then can be detected.

Still other amplification methods are described in GB Appl. No. 2 202 328, published September 21, 1988, and in PCT Appl. No. PCT/US89/01025, published October 5, 1989. In the former application, "modified" primers are used in a PCR like, template and enzyme dependent synthesis. The primers may be modified by labelling with a capture moiety (e.g., biotin) and/or a detector moiety (e.g., enzyme). In the latter application, an excess of labelled probes are added to a sample. In the presence of the target sequence, the probe binds and is cleaved catalytically. After cleavage, the target sequence is released intact to be bound by excess probe. Cleavage of the labelled probe signals the presence of the target sequence.

For all of the above-described methods, a variety of detection methods may be employed none of which are critical to the amplification method employed. One method is detect reaction products having a specific size via electrophoresis. Another method is to radiolabel the probe sequence with $^{32}$P, for example, and then to detect the radioactivity emitted by the reaction products alone or in combination with electrophoresis. A further method is to chemically modify the primer by adding a binding molecule (e.g., biotin), and enzyme (e.g., alkaline phosphatase), a fluorescent dye (e.g., phycobiliprotein) or a combination. Another method is to develop a detection primer which will bind to the reaction product and be extended in the presence of polymerase. The detection primer can be radiolabelled or chemically modified as described above. Many of these methods may be adapted to solid phase as well as solution systems. A number of these methods, as well as others, are described in U.S. Pat. No.s 4,358,535, 4,705,886, 4,743,535, 4,777,129, 4,767,699, and 4,767,700.

Each of the above-referenced amplification methods has one or more limitations. In most of the amplification methods, a key limitation is the requirement for temperature cycling to cause the reaction products to dissociate from the target. This places a limitation on both the devices used to perform the method as well as on the choice of enzymes necessary to form the reaction products. Other limitations of these methods include production of RNA intermediates sensitive to endogenous nuclease degradation and difficulty in production of associated enzymes. Alternative methods to such existing amplification methods are desirable.

Summary of the Invention

This invention provides for a method of amplification of a target nucleic acid sequence (and its complementary strand) in a sample by exonuclease mediated strand displacement. In its simplest form, the method comprises the steps of adding to a target nucleic acid sequence to be amplified a mixture comprising (a) a nucleic acid polymerase, (b) deoxynucleosidetriphosphates including at least one substituted deoxynucleosidetriphosphate, (c) an exonuclease and (d) at least one primer which is complementary to a region at the 3' end of a target fragment, and allowing the mixture to react for a time sufficient to generate reaction products. Where the nucleic acids are to be isolated from a sample comprising a biological material, the method further involves the initial steps of 1) isolating nucleic acids suspected of containing the target sequence from a sample, and 2) generating single stranded fragments of target sequences. Where the fragments comprise double standard nucleic acids, the method further comprises denaturing the nucleic acid fragments to form single stranded target sequences. Where the nucleic acids comprise RNA, it is preferable to use reverse transcriptase to convert RNA to DNA.

The invention further relates to methods for the separation and/or detection of reaction products generated by the above-described method. Methods for separation comprise magnetic separation, membrane capture and capture on solid supports. In each method, a capture moiety may be bound to a magnetic bead, membrane or solid support. The beads, membrane or solid support then can be assayed for the presence or absence of reaction products. An example of a capture moiety includes a nucleic sequence complementary to the reaction products produced and an antibody directed against a receptor incorporated into the primer or reaction product. The separation system may or may not be coupled to a detection system.

Detection systems useful in the practice of this invention comprise homogeneous systems, which do not require separation, and heterogeneous systems. In each system, one or more detectable markers are used and the reaction or emission from the detection system is monitored, preferably by automated means. Examples of homogeneous systems include fluorescence polarization, enzyme mediated immunoassays, fluorescence energy transfer, hybridization protection (e.g., acridinium luminescence) and cloned enzyme donor immunoassays. Examples of heterogeneous systems include enzyme labels (such as peroxidase, alkaline phosphatase and beta-galactosidase), fluorescent labels (such as enzymatic labels and direct fluorescence labels [e.g., fluorescein and rhodamine]), chemiluminescence and bioluminescence. Liposomes or other sac like particles also can be filled with dyes and other detectable markers and used in such detection systems. In these systems, the detectable markers can be conjugated directly or indirectly to a capture moiety or the reaction products can be generated in the presence of a receptor which can be recognized by a ligand for the receptor.

The invention still further relates to methods of generating reaction products which, by removing the recognition sequence at the 5' end, can function as probes. In this format, the above described method and steps are used to generate reaction products. The reaction products then may be treated with a restriction enzyme to cleave the recognition sequence from the reaction product. In this manner, the recognition sequence is removed and the remaining reaction product comprises a probe which can be used in other systems.

In the presence of a single stranded fragment, a primer will bind to its complementary target strand. In the presence of polymerase, nucleotides and substituted nucleotides will be added to the 3' end of the primer along the remaining length of the target to form a probe strand. The resulting double stranded product will comprise an unsubstituted target strand while the probe strand will comprise an unsubstituted primer coupled 5′ to an extended sequence complementary to the target sequence.

In the presence of a double strand specific 5′→3′ exonuclease, the primer portion of the probe will be digested up to the point of the first substituted nucleotide but no farther because its sequence contains the substituted nucleotides. A new primer then will bind to the single strand portion of the target. As it does so, it will be extended by the polymerase; however, the probe strand lacking the primer portion which is still bound to the target will be displaced by the polymerase downstream (i.e., 5′→3′) generating a reaction product that is complementary to the target strand. Thus, while the strand is displaced, the polymerase also will generate a new probe strand adding nucleotides to the primer.

The method also can function with two primers wherein one primer will bind to one strand of a target sequence and the other primer will bind to the complementary strand of the target sequence. When this embodiment is used, it will be apparent that each reaction product can function as a "target" for the other primer. In this manner, amplification proceeds logarithmically.

Brief Description of the Drawings

FIG. 1 comprises a flow chart of the steps in an example of the method claimed in this invention for a single stranded DNA fragment.

FIG. 2 comprises a flow chart of the steps in an example of the method claimed in this invention for double stranded genomic DNA.

Detailed Description

In this invention, the sample may be isolated from any biological material suspected of containing the target nucleic acid sequence. For animals, preferably, mammals, and more preferably humans, the sources of such materials may comprise blood, bone marrow, lymph, hard tissues (e.g., liver, spleen, kidney, lung, ovary, etc.), sputum, feces and urine. Other sources of material may be derived from plants, soil and other materials suspected of containing biological organisms.

The isolation of nucleic acids from these materials can be done any number of ways. Such methods include the use of detergent lysates, sonication, vortexing with glass beads and a French press. In some instances, it may be advantageous to purify the nucleic acids isolated (e.g., where endogenous nucleases are present). In those instances, purification of the nucleic acids may be accomplished by phenol extraction, chromatography, ion exchange, gel electrophoresis or density dependent centrifugation.

Once the nucleic acids are isolated, it will be assumed for purposes of illustration only that the genomic nucleic acid is DNA and is double stranded. In such instances, it is preferred to cleave the nucleic acids in the sample into fragments of between approximately 50-500bp. This may be done by a restriction enzyme such as Hha I, Fok I or Dpn I. The selection of the enzyme and the length of the sequence should be such so that the target sequence sought likely will be contained in its entirety within the fragment generated or at least a sufficient portion of the target sequence will be present in the fragment to provide sufficient binding of the probe sequence. Other methods for generating fragments include PCR and sonication.

The primers used in this method generally have a length of 15 - 100 nucleotides. Primers of approximately 20 nucleotides are preferred. This sequence should be substantially homologous to a sequence on the target such that under high stringency conditions binding will occur.

Once target nucleic acid fragments are generated, they are denatured to render them single stranded so as to permit binding of the primers to the target strands. Raising the temperature of the reaction to approximately 95°C is a preferred method for denaturing the nucleic acids. Other methods include raising pH; however, this will require lowering the pH in order to allow the probes to bind to the target.

Either before or after the nucleic acids are denatured, a mixture comprising an excess of all four deoxynucleosidetriphosphates wherein at least one of which is substituted, a polymerase and an exonuclease are added. (If high temperature is used to denature the nucleic acids, unless thermophilic enzymes are used, it is preferrable to add the enzymes after denaturation.) The substituted deoxynucleosidetriphosphate should be modified such that it will inhibit digestion of the strand containing the substituted deoxynucleotides. An example of such a substituted deoxynucleosidetriphosphates includes 2′deoxyadenosine 5′-O-(1-thiotriphosphate). The modified nucleotide could alternatively be incorporated into the primer during chemical synthesis. In this case, modified deoxynucleosidetriphosphates are not necessary.

The selection of the exonuclease used in this method should be such that it will digest double stranded DNA. The exonuclease further should be selected so as not to digest the target strand or the substituted portion of the probe. It need not be thermophilic. Exonucleases such as T7 gene 6 exonuclease and lamda exonuclease (U.S. Biochemical) are particularly useful.

Polymerases useful in this method include those that will polymerize the target from the 5′ end to the 3′ end. The polymerase, however, should also displace the polymerized target without digesting the template strand, and, importantly, should lack any 5′

to 3′ exonuclease activity. Polymerases such as the klenow fragment of DNA polymerase I and the exonuclease deficient klenow fragment of DNA polymerase I (United States Biochemical) and a similar fragment from the Bst polymerase (BioRad) are useful.

An additional feature of this method is that it need not be run a varying temperatures. Many amplification methods require temperature cycling in order to dissociate the target from the synthesized strand. In this method, a single temperature may be employed after denaturation has occurred. The temperature of the reaction should be high enough to set a level of stringency that minimizes non-specific binding but low enough to minimize the time within which binding of the probe to the target strand takes place. 37°C has been found to be a preferred temperature.

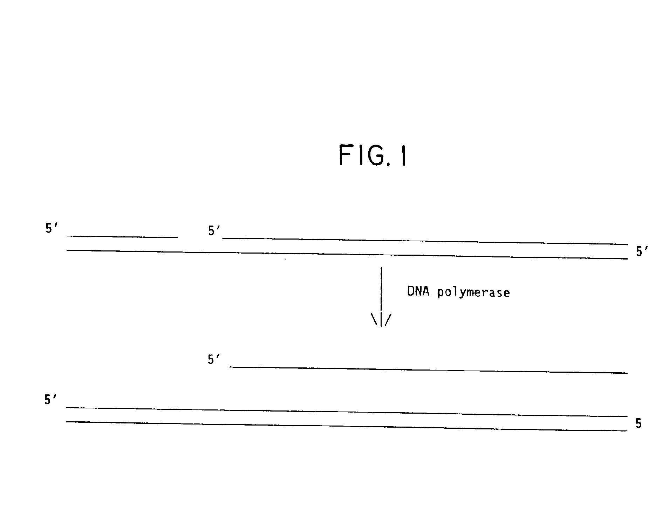

Referring to FIG. 1, one example of this invention is set forth. In this example, the strand labelled P represents the primer. The strand labelled T is the target sequence which has already been fragmented and rendered single stranded. In the method, the primer binds to the target and in the presence of polymerase, deoxynucleosidetriphosphates and αthio substituted deoxynucleosidetriphosphate, the primer is extended the length of the target. In the presence of the exonuclease, the probe strand is digested beginning at the 5′ end until it comes to the first substituted nucleotide. A new primer then binds to the target; however, in the presence of the polymerase lacking 5′→3′ exonuclease activity, the partially digested probe strand is displaced from the target strand beginning at the first substituted nucleotide to create a reaction product and a new strand is synthesized from the newly bound primer . In summary fashion (not shown), the newly synthesized strand too will be digested by the exonuclease and the polymerase then will displace this strand generating another until either the reaction is stopped or one of the reagents becomes limiting.

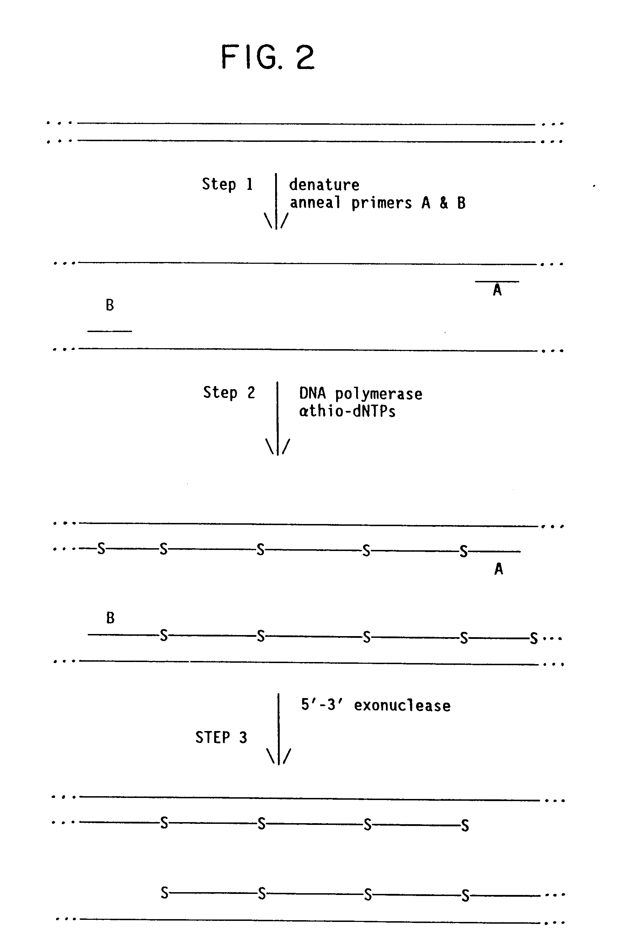

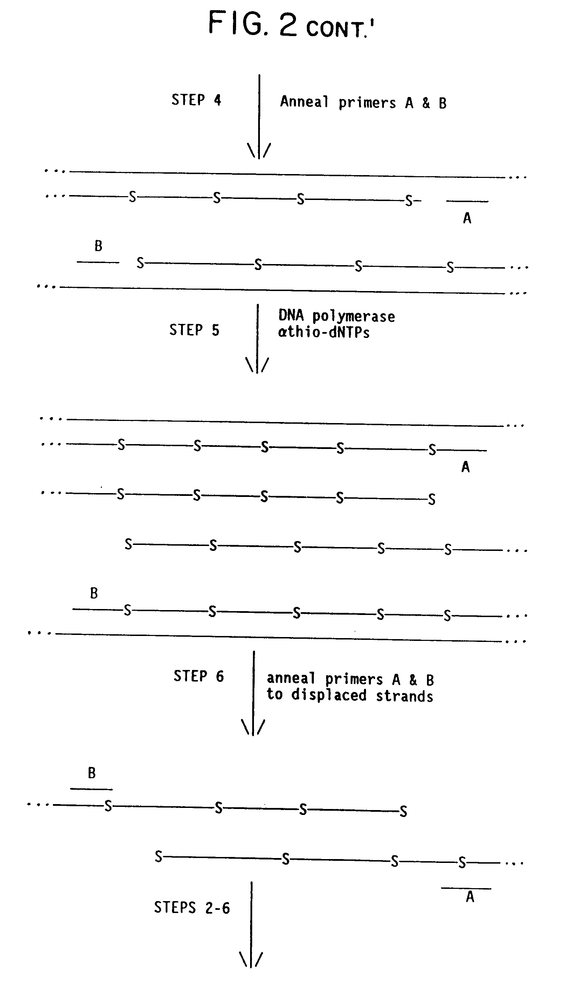

Referring to FIG. 2, an example is shown for a double stranded target. Thus, instead of having one primer, there are two primer ($P_1$ and $P_2$), one each being complementary to the two target sequences ($T_1$ and $T_2$). The reaction then proceeds as described for FIG. 1. It is to be noted, however, that $P_1$ will bind to the reaction product produced from $T_2$ while $P_2$ will bind to the reaction product produced from $T_1$. In this manner, amplification proceeds logarithmically.

The presence of the amplified target then can be detected by any number of methods. One method is to detect reaction products of a specific size by means of gel electrophoresis. This method is particularly useful when the nucleotides used are labelled with a radio-label, such as $^{32}P$. Other methods include the use of labelling the nucleotides with a physical label, such as biotin. Biotin containing reaction products can then be identified by means of avidin bound to a signal generating enzyme, such as peroxidase.

An additional embodiment of this invention is in the preparation of multiple copies of a single stranded nucleic acid sequence. It will be appreciated that the production of synthetic probes on a device like the Applied Biosystems 380 instrument is time consuming and is not readily applicable to generation of probes having several hundred nucleotides. The method of exonuclease mediated strand displacement need not be used to amplify a signal to detect the presence of a target for diagnostic purposes. It also is readily applicable to the generation of multiple copies of a single stranded nucleic acid sequence for use as a probe in any system of detection.

In this embodiment, a single stranded nucleic acid sequence to be amplified is prepared. This can be made from a synthetic sequence or genomic nucleic acids as described above. A reaction mixture similar to that describe above is prepared using a probe complementary to the nucleic acid sequence to be amplified. The reaction is then allowed to take place as above. The resulting reaction products will comprise the amplified copies of the nucleic acid sequence and can be used as primers or probes.

All publications and patent applications mentioned in this specification are indicative of the level of ordinary skill in the art to which this invention pertains. All publications and patent applications are herein incorporated by reference to the same extent as if each individual publication or patent application was specifically and individually indicated to be incorporated by reference.

It will be apparent to one of ordinary skill in the art that many changes and modifications can be made in the invention without departing from the spirit or scope of the appended claims.

## Claims

1. A method for amplifying a target nucleic acid sequence in a sample comprising the steps of:

a) isolating nucleic acid fragments from a sample;

b) preparing single stranded target nucleic acids;

c) adding to the target sequence sample a reaction mixture comprising an excess of deoxynucleosidetriphosphates, at least one of which is substituted, a DNA polymerase lacking 5′ to 3′ exonuclease activity, one or more primers that are complementary to the single strands of the target sequence, and a double strand specific 5′→3′ exonuclease;

d) allowing the reaction mixture to react with the single stranded fragments for a period of time sufficient to generate reaction products; and

e) detecting the presence of the reaction products produced.

2. The method of claim 1 wherein a restriction enzyme is used to create nucleic acid fragments.

3. The method of claim 1 wherein the sample is derived from animal material comprising blood, bone marrow, lymph, hard tissue, urine, feces and sputum, plant material and soil.

4. The method of claim 1 wherein the nucleic and fragments are rendered single stranded by heating the sample.

5. A method for amplifying a target nucleic acid sequence comprising the steps of:
   a) isolating nucleic acids from a sample wherein the sample is derived from human biological material;
   b) generating single stranded nucleic acids by heating the sample;
   c) adding a reaction mixture comprising an excess of deoxynucleosidetriphosphates at least one of which is substituted, a DNA polymerase lacking 5′ to 3′ exonuclease activity, one or more primers that are complementary to the single strands of the target sequence, and a double strand specific 5′→3′ exonuclease;
   d) allowing the reaction mixture to react with the single stranded nucleic acids for a period of time sufficient to generate reaction products; and
   e) detecting the reaction products produced

6. The method of claim 5 wherein the polymerase is selected from the group consisting of the klenow fragment of DNA polymerase I, the exonuclease deficient klenow fragment of DNA polymerase I and the klenow fragment of <u>Bst</u> polymerase.

7. The method of claim 6 wherein the polymerase is the klenow fragment of DNA polymerase I.

8. The method of claim 5 wherein the exonuclease is T7 gene 6 exonuclease.

9. The method of claim 5 wherein detection of the reaction products is carried out by tagging the primer with a label selected from the group consisting of a radiolabel, an enzyme and a fluorescent dye.

10. The method of any of claims 1 and 5 wherein the probes are specific for a viral nucleic acid sequence.

11. The method of any of claims 1 and 5 wherein the probes are specific for a bacterial nucleic acid sequence.

12. The method of any of claims 1 and 5 wherein the probes are specific for an oncogene nucleic acid sequence.

13. The method of any of claims 1 and 5 wherein the probes are specific for a nucleic acid sequence involved in a single point mutation.

# FIG. 1

DNA polymerase

# FIG. 2

Step 1 | denature
anneal primers A & B

A

B

Step 2 | DNA polymerase
αthio-dNTPs

—S——S———S———S———S—
A

B
———S———S———S———S——S

5'-3' exonuclease

STEP 3

S———S———S———S

S———S———S———S

# FIG. 2 CONT.'